# EUROPEAN PATENT APPLICATION

(11) **EP 4 570 243 A1**
(43) Date of publication of application: **18.06.2025**
(21) Application number: 24218952.0
(22) Date of filing: 11.12.2024
(51) Int. Cl.: A61K 9/46, A61K 31/4015

(54) **EFFERVESCENT TABLET FORMULATION OF BRIVARACETAM**

(30) Priority: 13.12.2023 TR 202317169
(71) Applicant: Sanovel Ilac Sanayi Ve Ticaret Anonim Sirketi, Istanbul (TR)
(72) Inventor: SUNEL, Fatih, Istanbul (TR); BILGEHAN ATAK, Fadime, Istanbul (TR); ATAMAN, Seval, Istanbul (TR); DERELI, Selin, Istanbul (TR)
(74) Representative: Genç Ilhan, Oznur

(57) **Abstract**

The present invention relates to an effervescent tablet formulation comprising brivaracetam or a pharmaceutically acceptable salt thereof, and at least one acid source and at least one gas generating agent, wherein the amount of brivaracetam is between 0.1% and 6.0% by weight in the total formulation. The present invention also relates to a simple, rapid, cost effective, time-saving and industrially convenient process of preparing the effervescent tablet.

## Description

### Field of the Invention

The present invention relates to an effervescent tablet formulation comprising brivaracetam or a pharmaceutically acceptable salt thereof, and at least one acid source and at least one gas generating agent, wherein the amount of brivaracetam is between 0.1% and 6.0% by weight in the total formulation. The present invention also relates to a simple, rapid, cost effective, time-saving and industrially convenient process of preparing the effervescent tablet.

### Background of the Invention

The chemical name of brivaracetam is (2S)-2-[(4R)-2-oxo-4-propyl-pyrrolidin-1-yl] butanamide and its chemical structure is shown in the Formula I.

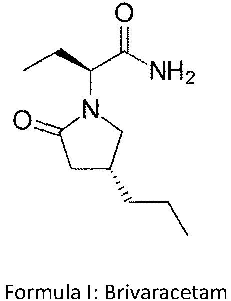

Brivaracetam is a white to off-white crystalline powder. It is very soluble in water, buffer (pH 1.2, 4.5 and 7.4), ethanol, methanol, and glacial acetic acid. It is freely soluble in acetonitrile and acetone and soluble in toluene. It is very slightly soluble in n-hexane. Additionally, brivaracetam is an extremely viscous compound (adhesive capacity).

Brivaracetam is commercially approved in the form of tablets, oral solution and injection, in the USA under the brand name Briviact and are marketed by UCB Pharma.

Effervescent tablets are designed to break down quickly and release carbon dioxide when dropped in liquid. They have gained considerable attention as a preferred alternative to conventional tablets and capsules due to their better patient compliance. Today, there are a growing number of people who cannot swallow tablets or capsules. On the other side, in effervescent tablet formulations the active ingredient is already solubilized before its administration and liquid effervescent form is easier to take as compared to tablets or capsules.

Taking big tablets or capsules is difficult for the patients. Effervescent technology provides an alternative to them. Dissolving and break-down of standard tablets also takes additional time in the stomach. In effervescents, ingredients are distributed in the solution and they are not localized in certain point. They can be taken in liquids and promotes patients to take more liquid. Absorption is improved and usage is easy in effervescent tablets.

The patent EP3799864A1 related an effervescent solid pharmaceutical composition which is free of sodium and which has a fast disintegration upon contact with water before its administration.

There still remains a need in the prior art to provide an improved pharmaceutical effervescent tablet formulation comprising brivaracetam or pharmaceutically acceptable salts thereof.

There are no effervescent tablets containing brivaracetam in the prior art. Brivaracetam has low melting point that result in some challenges in tablet coating such as pitting of the coating layer. Since the use of effervescent does not contain a coating, it has become advantageous in terms of physical stability as well as patient compliance.

We have found that Brivaracetam is extremely prone to sticking and picking due to high adhesion during the preparation process, which brings great problems to the appearance and content uniformity of the final product. Also, during the process, problems such as flowability and compressibility appear. These problems lead to both solubility and stability problems.

### Detailed Description of the Invention

The main object of the present invention provides an effervescent tablet formulation comprising brivaracetam having the desired stability and high disintegration time.

Another object of the present invention is to obtain an effervescent tablet formulation comprising brivaracetam having content uniformity.

Another object of the present invention is to provide a preparation process of the effervescent tablet formulation comprising brivaracetam which is simple and cost-effective process.

The high adhesion property of brivaracetam causes some undesirable problems, one of which is content uniformity. In order to overcome the sticking feature of brivaracetam, we achieved the desired content uniformity by using brivaracetam in the following proportions in the effervescent formulation. In this way, the desired dissolution profile and stability were achieved.

The present invention an effervescent tablet formulation comprising brivaracetam or a pharmaceutically acceptable salt thereof, and at least one acid source and at least one gas generating agent, wherein the amount of brivaracetam is between 0.1% and 6.0% by weight in the total formulation.

Preferably, the amount of brivaracetam is between 0.5% and 5.0% by weight in the total formulation.

Suitable acid sources are selected from the group comprising citric acid anhydrous, nicotinic acid, dilute hydrochloric acid, glacial acetic acid, malic acid, ascorbic acid, acetylsalicylic acid, lactic acid, maleic acid, acid, adipic acid, tartaric acid or mixtures thereof.

According to this embodiment of the present invention, the amount of acid source is between 5.0% and 35.0% by weight in the total formulation. Preferably, the amount of acid source is between 10.0% and 25.0% by weight in the total formulation.

According to this embodiment of the present invention, the acid source is citric acid anhydrous.

We chose citric acid as the acid source because citric acid imparts a pleasant citrus-type taste and acts as a flavour enhancer, making the formulation easier to drink with less sweetener.

Suitable gas generating agents are selected from the group comprising sodium bicarbonate, sodium carbonate anhydrous, calcium carbonate, aluminum potassium sulfate, anhydrous disodium hydrogen phosphate, potassium bicarbonate, anhydrous sodium dihydrogen phosphate, dibasic potassium sulfate, monobasic potassium phosphate, sodium acetate, sodium carbonate, sodium glycine carbonate sodium citrate, sodium dihydrogen phosphate dihydrate, tribasic sodium phosphate or mixtures thereof.

According to this embodiment of the present invention, the amount of gas generating agent is between 1.0% and 35.0% by weight in the total formulation. Preferably, the amount of gas generating agent is between 2.0% and 25.0% by weight in the total formulation.

According to an embodiment of the present invention, the gas generating agent is sodium bicarbonate or sodium carbonate anhydrous or calcium carbonate or mixtures thereof.

Gas generating in effervescent tablet formulations is obtained due to the presence of acid and base substances such as carbonates or bicarbonates that react rapidly in the presence of water and release C0₂ to provide a carbonated or sparkling liquid drink. Due to liberation of C0₂, the dissolution of the API in water also helps to the masking of the taste of the API.

According to an embodiment of the present invention, the effervescent tablet formulations further comprise at least one pharmaceutically acceptable excipient which is selected from the group comprising diluent, binder, sweetener, flavouring agent, lubricant or mixtures thereof.

Suitable diluents are selected from the group comprising mannitol, lactose anhydrous, lactose monohydrate, dextrose, starch, fructose, maltose, xylitol, inositol, kaolin, inorganic salts, calcium salts, polysaccharides, dicalcium phosphate, sodium chloride, dextrates, lactitol, microcrystalline cellulose or mixtures thereof.

According to this embodiment of the present invention, the amount of diluent is between 1.0% and 70.0% by weight in the total formulation. Preferably, the amount of diluent is between 5.0% and 60.0% by weight in the total formulation. These ratios helped to provide the content uniformity.

According to an embodiment of the present invention, the diluent is mannitol or lactose anhydrous or mixtures thereof.

Suitable binders are selected from the group comprising polyvinylpyrrolidone, hydroxypropyl cellulose, sodium carboxymethyl cellulose, sodium carboxymethyl cellulose, polyethylene glycol, starch, pregelatinized starch, sodium alginate, hydroxypropyl methyl cellulose, carboxy methyl cellulose, methyl cellulose, guar gum, polymethacrylates, methacrylate polymers, polysaccharides, poloxamer, polyoxyethylene-alkyl ether, polydextrose, polyethylene oxide or mixtures thereof.

According to this embodiment of the present invention, the amount of binder is between 0.1% and 8.0% by weight in the total formulation. Preferably, the amount of binder is between 0.5% and 5.0% by weight in the total formulation.

According to an embodiment of the present invention, the binder is polyvinylpyrrolidone.

Suitable sweeteners are selected from the group comprising sucralose, aspartame, sorbitol, erythritol, thaumatin, mogroside, inulin, acesulfame-K, saccharin or its sodium and calcium salts, sodium cyclamate, sucrose, glucose, menthol, peppermint, cinnamon or mixtures thereof.

According to this embodiment of the present invention, the amount of sweetener is between 0.1% and 10.0% by weight in the total formulation. Preferably, the amount of sweetener is between 0.5% and 6.0% by weight in the total formulation.

According to an embodiment of the present invention, the sweeteners is sucralose or aspartam or sorbitol or mixtures thereof.

Suitable flavouring agents are selected from the group comprising orange flavor, cherry flavor, lemon flavor, menthol, peppermint, cinnamon, chocolate, vanillin, strawberry, grape, black currant, raspberry, banana, red fruits, wild berries or mixtures thereof.

According to this embodiment of the present invention, the amount of flavouring agent is between 0.05% and 10.0% by weight in the total formulation. Preferably, the amount of flavouring agent is between 0.1% and 7.0% by weight in the total formulation.

According to an embodiment of the present invention, the flavouring agent is orange flavor or cherry flavor or mixtures thereof.

Suitable lubricants are selected from the group comprising sodium benzoate, sodium stearyl fumarate, magnesium stearate, sodium lauryl sulphate, zinc stearate, calcium stearate, mineral oil, polyethylene glycol, glyceryl monostearate, glyceryl palmitostearate, magnesium lauryl sulphate, fumaric acid, stearic acid, hydrogenated natural oils, silica, paraffin or mixtures thereof.

According to this embodiment of the present invention, the amount of lubricant is between 0.1% and 8.0% by weight in the total formulation.

According to an embodiment of the present invention, the lubricant is sodium benzoate or sodium stearyl fumarate.

### Example 1;

| **Ingredients** | **% by weight** | **% by weight** | **% by weight** |
|---|---|---|---|
| Brivaracetam | 1 | 2.5 | 0.1-6 |
| Lactose Anhydrous | 38.25 | 36.75 | 25-45 |
| Sitric acid (anhydrous) | 22 | 22 | 10-30 |
| Sodium bicarbonate | 19.6 | 19.6 | 10-25 |
| Sodium carbonate (anhydrous) | 4.4 | 4.4 | 1-10 |
| Mannitol | 9.5 | 9.5 | 1-20 |
| Aspartam | 2.0 | 2.0 | 0.1-6 |
| Orange Flavor | 0.75 | 0.75 | 0.05-5 |
| Sodium benzoate | 2.5 | 2.5 | 0.1-6 |
| **TOTAL TABLET** | **100** | **100** | **100** |

### Method of Manufacture

1) Weigh and sieve Brivaracetam, Lactose anhydrous, citric acid, sodium bicarbonate, sodium carbonate, mannitol, aspartam, orange flavor and mix for 15 minutes.
2) Add weighed and 0.6 mm sieved sodium benzoate to dry mixture and mix for 3 minutes
3) Tablet the homogenous mixture following appropriate specifications

### Example 2;

| **Ingredients** | **% by weight** | **% by weight** | **% by weight** |
|---|---|---|---|
| Brivaracetam | 1 | 2.5 | 0.1-6 |
| Mannitol | 51.5 | 50 | 40-60 |
| Citric acid anyhdrous | 15 | 15 | 5-25 |
| Sodium bicarbonate | 20 | 20 | 5-35 |
| Sucralose | 4.5 | 4.5 | 0.5-10 |
| Orange flavor | 5 | 5 | 0.5-10 |
| Sodium benzoate | 3 | 3 | 0.1-8 |
| **TOTAL TABLET** | **100** | **100** | **100** |

### Method of Manufacture

1) Weigh and sieve Brivaracetam, citric acid, sodium bicarbonate, mannitol, sucralose, orange flavor and mix for 15 minutes.
2) Add weighed and 0.6 mm sieved sodium benzoate to dry mixture and mix for 3 minutes
3) Tablet the homogenous mixture following appropriate specifications.

### Example 3;

| **Ingredients** | **% by weight** | **% by weight** | **% by weight** |
|---|---|---|---|
| Brivaracetam | 1 | 2,5 | 0.1-6 |
| Citric acid anhydrous | 20 | 20 | 5-35 |
| Calcium Carbonate | 15 | 15 | 5-25 |
| Lactose anhydrous | 58 | 56.5 | 40-70 |
| PVP | 1.5 | 1.5 | 0.1-6 |
| Sodium Stearyl Fumarate | 1.5 | 1.5 | 0.1-6 |
| Sucrose | 1.5 | 1.5 | 0.1-6 |
| Cherry flavor | 1.5 | 1.5 | 0.1-6 |
| **TOTAL TABLET** | **100** | **100** | **100** |

### Method of Manufacture

1) Weigh and sieve Brivaracetam, Lactose anhydrous, citric acid, calcium carbonate, PVP, sucrose, cherry flavor and mix for 15 minutes.
2) Add weighed and 0.6 mm sieved sodium stearyl fumarate to dry mixture and mix for 3 minutes
3) Tablet the homogenous mixture following appropriate specification.

### Example 4;

| **Ingredients** | **% by weight of the total tablet core** | **% by weight of the total tablet core** | **% by weight of the total tablet core** |
|---|---|---|---|
| Brivaracetam | 1 | 2.5 | 0.1-6 |
| Lactose Anhydrous | 34.7 | 33.2 | 15-50 |
| PVP | 2.6 | 2.6 | 0.1-8 |
| Mannitol | 9 | 9 | 1-20 |
| Sorbitol | 6.9 | 6.9 | 0.5-15 |
| Citric acid anhydrous | 20 | 20 | 5-35 |
| Sodium bicarbonate | 20 | 20 | 5-35 |
| Aspartame | 1.9 | 1.9 | 0.1-6 |
| Orange flavor | 0.9 | 0.9 | 0.1-6 |
| Sodium benzoate | 3 | 3 | 0.1-8 |
| **TOTAL TABLET** | **100** | **100** | **100** |

### Method of Manufacture

1) Weigh and sieve Brivaracetam, lactose anhydrous, PVP, and mix for 15 minutes.
2) Add weighed and 0.6 mm sieved 1/3 sodium benzoate to dry mixture and mix for 3 minutes
3) Perform slug granulation in compactor
4) Sieve dry granules 1.2 mm
5) Add 0.6mm sieved mannitol, sorbitol, citric acid, sodium bicarbonate aspartame, orange flavor and mix for 10 minutes.
6) Add 0.6mm sieved 2/3 sodium benzoate and mix for 3 minutes
7) Tablet the homogenous mixture following appropriate specifications

## Claims

1. An effervescent tablet formulation comprising brivaracetam or a pharmaceutically acceptable salt thereof, and at least one acid source and at least one gas generating agent, wherein the amount of brivaracetam is between 0.1% and 6.0% by weight in the total formulation.

2. The effervescent tablet formulation according to claim 1, wherein acid sources are selected from the group comprising citric acid anhydrous, nicotinic acid, dilute hydrochloric acid, glacial acetic acid, malic acid, ascorbic acid, acetylsalicylic acid, lactic acid, maleic acid, acid, adipic acid, tartaric acid or mixtures thereof.

3. The effervescent tablet formulation according to claim 2, wherein the acid source is citric acid anhydrous.

4. The effervescent tablet formulation according to claim 1, wherein gas generating agents are selected from the group comprising sodium bicarbonate, sodium carbonate anhydrous, calcium carbonate, aluminum potassium sulfate, anhydrous disodium hydrogen phosphate, potassium bicarbonate, anhydrous sodium dihydrogen phosphate, dibasic potassium sulfate, monobasic potassium phosphate, sodium acetate, sodium carbonate, sodium glycine carbonate sodium citrate, sodium dihydrogen phosphate dihydrate, tribasic sodium phosphate or mixtures thereof.

5. The effervescent tablet formulation according to claim 4, wherein the gas generating agent is sodium bicarbonate or sodium carbonate anhydrous or calcium carbonate or mixtures thereof.

6. The effervescent tablet formulation according to claim 1, wherein the effervescent tablet formulations further comprise at least one pharmaceutically acceptable excipient which is selected from the group comprising diluent, binder, sweetener, flavouring agent, lubricant or mixtures thereof.

7. The effervescent tablet formulation according to claim 6, wherein diluents are selected from the group comprising mannitol, lactose anhydrous, lactose monohydrate, dextrose, starch, fructose, maltose, xylitol, inositol, kaolin, inorganic salts, calcium salts, polysaccharides, dicalcium phosphate, sodium chloride, dextrates, lactitol, microcrystalline cellulose or mixtures thereof.

8. The effervescent tablet formulation according to claim 7, wherein the diluent is mannitol or lactose anhydrous or mixtures thereof.

9. The effervescent tablet formulation according to claim 6, wherein binders are selected from the group comprising polyvinylpyrrolidone, hydroxypropyl cellulose, sodium carboxymethyl cellulose, sodium carboxymethyl cellulose, polyethylene glycol, starch, pregelatinized starch, sodium alginate, hydroxypropyl methyl cellulose, carboxy methyl cellulose, methyl cellulose, guar gum, polymethacrylates, methacrylate polymers, polysaccharides, poloxamer, polyoxyethylene-alkyl ether, polydextrose, polyethylene oxide or mixtures thereof.

10. The effervescent tablet formulation according to claim 9, wherein the binder is polyvinylpyrrolidone.

11. The effervescent tablet formulation according to claim 6, wherein sweeteners are selected from the group comprising sucralose, aspartame, sorbitol, erythritol, thaumatin, mogroside, inulin, acesulfame-K, saccharin or its sodium and calcium salts, sodium cyclamate, sucrose, glucose, menthol, peppermint, cinnamon or mixtures thereof.

12. The effervescent tablet formulation according to claim 11, wherein the sweeteners is sucralose or aspartam or sorbitol or mixtures thereof.

13. The effervescent tablet formulation according to claim 6, wherein flavouring agents are selected from the group comprising orange flavor, cherry flavor, lemon flavor, menthol, peppermint, cinnamon, chocolate, vanillin, strawberry, grape, black currant, raspberry, banana, red fruits, wild berries or mixtures thereof.

14. The effervescent tablet formulation according to claim 6, wherein lubricants are selected from the group comprising sodium benzoate, sodium stearyl fumarate, magnesium stearate, sodium lauryl sulphate, zinc stearate, calcium stearate, mineral oil, polyethylene glycol, glyceryl monostearate, glyceryl palmitostearate, magnesium lauryl sulphate, fumaric acid, stearic acid, hydrogenated natural oils, silica, paraffin or mixtures thereof.

15. The effervescent tablet formulation according to claim 14, wherein the lubricant is sodium benzoate or sodium stearyl fumarate.
